Europäisches Patentamt

European Patent Office

Office européen des brevets

(11) Veröffentlichungsnummer: **0 047 985**
**A1**

(12) # EUROPÄISCHE PATENTANMELDUNG

(21) Anmeldenummer: **81107139.8**

(22) Anmeldetag: **10.09.81**

(51) Int. Cl.³: **A 61 M 25/00**

(30) Priorität: **12.09.80 DE 8024412 U**

(43) Veröffentlichungstag der Anmeldung:
**24.03.82 Patentblatt 82/12**

(84) Benannte Vertragsstaaten:
**AT BE CH DE FR GB IT LI LU NL SE**

(71) Anmelder: **INTERMEDICAT GMBH**
**Gerliswilstrasse 43**
**CH-6020 Emmenbrücke(CH)**

(72) Erfinder: **Herlitze, Gerhard**
**Binsdorfer Strasse 4**
**D-3507 Baunatal 7(DE)**

(74) Vertreter: **von Kreisler, Alek, Dipl.-Chem. et al,**
**Deichmannhaus am Hauptbahnhof**
**D-5000 Köln 1(DE)**

(54) **Venenverweilkapillare mit Fenster.**

(57) Eine Venenverweilkapillare (1) aus flexiblem Kunststoff mit röntgenpositiven Bestandteilen weist einen in Längsrichtung verlaufenden, nicht röntgenpositiven transparenten Festerstreifen (2) auf.

Fig. 1

EP 0 047 985 A1

VON KREISLER   SCHÖNWALD   EISHOLD   FUES
VON KREISLER   KELLER   SELTING   WERNER

**PATENTANWÄLTE**

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

DEICHMANNHAUS AM HAUPTBAHNHOF
**D-5000 KÖLN 1**

0047985

B.Braun Melsungen Aktiengesellschaft
3508 Melsungen

— 7 —

9.Sept.81   AvK/IM

Venenverweilkapillare mit Fenster
------------------------------------------------

Die Erfindung betrifft Fenster-Kapillaren oder windowall-
Kapillaren für Venenverweilkanülen.

Flüssigkeiten wie z.B. Infusionslösungen, Injektabilia
oder Blut werden in das Venensystem entweder über Venenkatheter oder Venenverweilkanülen, auch Kurzkatheter genannt, eingebracht. Die heute bekannten Venenverweilkanülen sind entweder einteilig  im Spritzgießverfahren oder
zweiteilige aus unterschiedlichen Materialien hergestellt.
Der Kapillarschlauch der zweiteiligen Venenverweilkanüle
ist entweder transparent oder röntgenpositiv. Röntgenpositive Kapillare haben den Nachteil, daß das durch -
strömende Medium von außen nicht sichtbar ist. Insbesondere ist der Rückfluss von Blut ein für den Anwender
leicht zu beurteilendes Merkmal für die erfolgreiche
Punktion mit der Venenverweilkanüle, wobei die Metallkanüle teilweise in das Kapillar zurückgezogen wird.
Dieser eben geschilderte Nachteil ist zwar bei transparenten Kapillarschläuchen nicht gegeben,jedoch sollten alle in
die Vene eingeführten Katheter oder Kapillare aus Sicherheitsgründen mit Röntgenkontraststreifen versehen sein.

Der Erfindung liegt die Aufgabe zugrunde, den Vorteil der
guten röntgenologischen Darstellbarkeit mit dem Vorteil einer
guten Sichtbarkeit des Blutrückflusses zu verbinden.

Die gestellte Aufgabe wird dadurch gelöst, daß die Kunststoffkapillare der Venenverweilkanüle auf mindestens der Hälfte
des Umfanges mit einem röntgenkontrastgebenden Mittel versetzt ist und der verbleibende Teil des Umfangs aus einem
transparenten, nicht röntgenpositiven Fensterstreifen besteht.
Dadurch sind einerseits eine im Bedarfsfalle röntgenologische
Darstellbarkeit in gewünschter Weise möglich und andererseits durch den transparenten Teil des Umfanges ein noch
guter Einblick in das Lumen gewährleistet.

Dementsprechend betrifft die Erfindung eine. Venenverweilkapillare aus flexiblem Kuntstoff mit röntgenpositiven Bestandteilen, gekennzeichnet durch einen in Längsrichtung
verlaufenden, nicht röntgenpositiven, transparenten Fensterstreifen.

Der flexible Kunststoffschlauch, aus dem die Venenverweilkapillare besteht, enthält auf mindestens der Hälfte seines
Umfanges ein röntgenpositives oder röntgenkontrastgebendes
Mittel. Der Teil des flexiblen Kunststoffschlauchs, der
kein Röntgenkontrastmittel enthält, ist transparent und bildet einen durchsichtigen Fensterstreifen.

Dieser Fensterstreifen beträgt 15 - 180$^{\circ}$, vorzugsweise 60
bis 120$^{\circ}$ des Umfangs des flexiblen Kunststoffschlauches,
aus dem die Venenverweilkapillare besteht.

Der Fensterstreifen ist in der Venenverweilkapillare so angeordnet, daß er für den Anwender der Venenverweilkapillare
einsehbar ist. Dies bedeutet, daß er auf der dem Anwender
zugewandten Seite der Venenverweilkapillare liegt.

In der Regel werden alle Venenverweilkapillaren am distalen Ende angeschrägt oder angeformt. Dadurch ergibt sich ein vom distalen Ende der Kapillare schräger Verlauf der Kapillare.

Bei einer besonderen Ausführungsform der Erfindung weist die angeschrägte oder angeformte Kapillarspitze eine wendelförmige Ausbildung des Fensterstreifens auf. Durch thermisches Anformen erhält die Spitze der Kapillare einen wendelförmigen Verlauf des Fensterstreifens. Dadurch wird ein weiterer Vorteil erreicht, der darin liegt, daß jederzeit ohne Hilfsmittel eine überprüfbare Qualitätsgarantie für einen atraumatischen stufenlosen Übergang zur Stahlkanüle gegeben ist.

Die Erfindung wird im Folgenden unter Bezugnahme auf die Zeichnungen weiter beschrieben.

Fig. 1    zeigt die Venenverweilkanüle mit der Kunststoffkapillaren in Teillänge und der innen befindlichen Stahlkanüle;

Fig. 2    zeigt eine Teilansicht der Venenverweilkanüle mit dem proximalen Ende der Kunststoffkapillaren;

Fig. 3    zeigt einen Schnitt durch Kunststoffkapillare und Stahlkanüle entlang der Schnittlinie A - A derFig. 1.

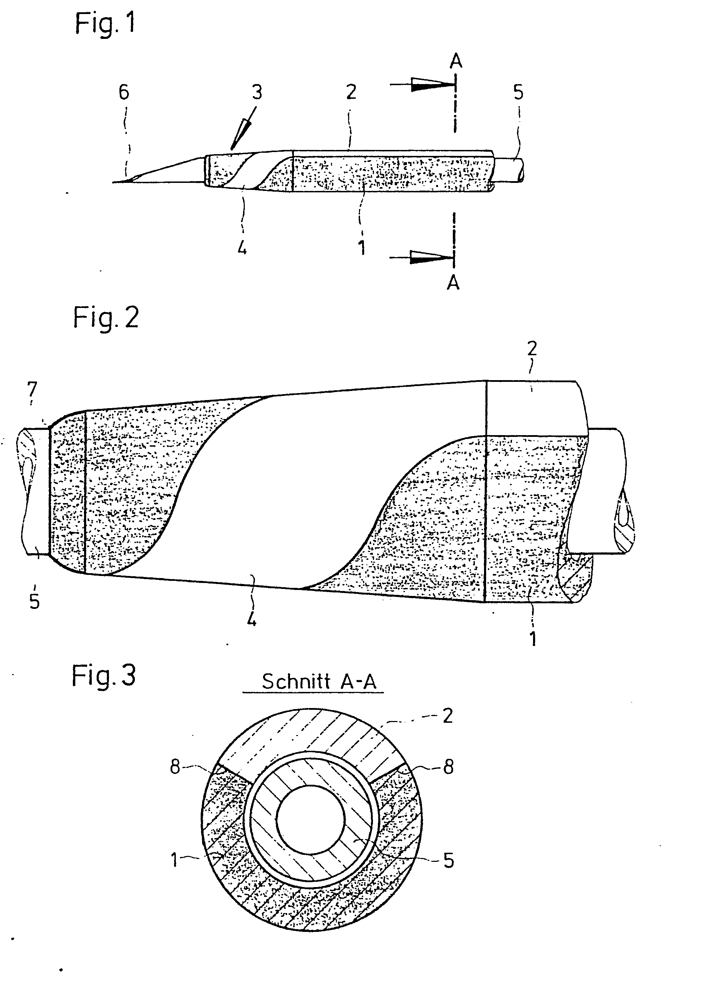

Fig. 1 zeigt die Kunststoffkapillare 1 mit dem in Längsrichtung verlaufenden Fensterstreifen 2, der am distalen Ende 3 der Kunststoffkapillaren einen spiralförmigen Verlauf 4 annimmt, sowie der innen liegenden Stahlkanüle 5 mit dem zur Venenpunktion geeigneten Schliff 6.

Fig. 2 zeigt den aus Längsrichtung in wendelförmigen Verlauf 4 übergehenden Fensterstreifen 2 mit der sich zum distalen Ende hin verjüngenden Kunststoffkapillaren 1 und dem auf der Stahlkanüle 5 auslaufenden stufenlosen Übergang 7.

Fig. 3 zeigt einen Schnitt durch die Stahlkanüle 5 mit der umhüllenden Kunststoffkapillaren 1 sowie dem mit dem restlichen Werkstoff der Kunststoffkapillaren im homogenen Verbund 8 befindlichen Fensterstreifen 2.

A n s p r ü c h e
-------------------

1.      Venenverweilkapillare aus flexiblem Kunststoff mit
röntgenpositiven Bestandteilen, gekennzeichnet durch einen
in Längsrichtung verlaufenden, nicht röntgenpositiven
transparenten Fensterstreifen (2).

2.      Venenverweilkapillare nach Anspruch 1, dadurch gekennzeichnet, daß der Fensterstreifen (2) 15 bis 180$^O$,
vorzugsweise 6o bis 12o$^O$ des Umfanges betärgt.

3.      Venenverweilkapillare nach Ansprüchen 1 und 2,
dadurch gekennzeichnet, daß der Fensterstreifen (2) in
der für den Anwender einsehbaren Lage angeordnet ist.

4.      Venenverweilkapillare nach Ansprüchen 1-3, dadurch gekennzeichnet, daß die Kapillarspitze (3) eine
wendelförmige Ausbildung (4) des Fensterstreifens (2)
aufweist.

Fig. 1

Fig. 2

Fig. 3

Schnitt A-A

Europäisches
Patentamt

**EUROPÄISCHER RECHERCHENBERICHT**

| EINSCHLÄGIGE DOKUMENTE | | | KLASSIFIKATION DER ANMELDUNG (Int. Cl ³) |
|---|---|---|---|
| Kategorie | Kennzeichnung des Dokuments mit Angabe, soweit erforderlich, der maßgeblichen Teile | betrifft Anspruch | |
| X | US - A - 3 529 633 (V.L. VAILLANCOURT) <br> * Ansprüche 1, 5, 6; Zusammenfassung; Spalte 1, Zeilen 23 bis 26, 44 bis 52; Fig. 1, 3 * <br> -- | 1-3 | A 61 M 25/00 |
| | US - A - 3 190 290 (R.D. ALLEY et al.) <br> * Anspruch 4, Spalte 1, Zeilen 22 bis 31 * <br> -- | 1,3 | |
| | DE - U1 - 7 824 519 (VYGON) <br> * Anspruch 1; Seite 3, Zeilen 20 bis 25; Seite 4, Zeilen 1 bis 4 * <br> -- | 1,3 | **RECHERCHIERTE SACHGEBIETE (Int. Cl.³)** |
| A | WO - A1 - 80/00061 (GENERAL ELECTRIC CO.) <br> * Ansprüche 1, 10 * <br> -- | 1,3 | A 61 M 25/00 <br> F 16 L 11/12 |
| A | FR - A - 2 187 363 (JOHNSON & JOHNSON) <br> * Seite 1, Zeilen 10 bis 23 * <br> & DE - A - 2 328 261 <br> -- | 1 | |
| A | US - A- 2 227 682 (WADE) <br> * Seite 4, Zeilen 12 bis 17; Fig. 3 * <br> -- | 1 | **KATEGORIE DER GENANNTEN DOKUMENTE** <br> X: von besonderer Bedeutung <br> A: technologischer Hintergrund <br> O: nichtschriftliche Offenbarung <br> P: Zwischenliteratur |
| A | US - A - 3 618 614 (V.J. FLYNN) <br> * Anspruch 1 * <br> --- <br> ./.. | 1 | T: der Erfindung zugrunde liegende Theorien oder Grundsätze <br> E: kollidierende Anmeldung <br> D: in der Anmeldung angeführtes Dokument <br> L: aus andern Gründen angeführtes Dokument |
| | | | &: Mitglied der gleichen Patent- familie, übereinstimmendes Dokument |

Der vorliegende Recherchenbericht wurde für alle Patentansprüche erstellt.

| Recherchenort | Abschlußdatum der Recherche | Prüfer |
|---|---|---|
| Berlin | 20-11-1981 | CLOT |

EPA form 1503.1 06.78

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US – A – 3 901 829 (E.L. SLINGLUFF et al.) <br><br> * Zusammenfassung * <br><br> –– | 1 |
| P | EP – A2 – 0 033 659 (TELEFLEX INCORPO-RATED) <br><br> * Ansprüche 1 bis 3, 9 bis 10; <br> Seite 2, Zeilen 1 bis 19; Seite 7, <br> Zeilen 21 bis 29; Seite 8, <br> Zeilen 1 bis 5; Fig. 3 * <br><br> ––––– | 1,3,4 |

KLASSIFIKATION DER
ANMELDUNG (Int. Cl.³) ·

RECHERCHIERTE
SACHGEBIETE (Int. Cl.³)